# EUROPEAN PATENT SPECIFICATION

(11) **EP 3 986 370 B1**
(45) Date of publication and mention of the grant of the patent: **29.10.2025**
(21) Application number: 20733928.4
(22) Date of filing: 16.06.2020
(51) Int. Cl.: A61K 8/89, A61K 8/44, A61K 8/46, A61K 8/73, A61K 8/81, A61Q 5/02, A61Q 5/12

(54) **PERSONAL CLEANSING COMPOSITIONS**
KÖRPERREINIGUNGSZUSAMMENSETZUNGEN
COMPOSITIONS D'HYGIÈNE PERSONNELLE

(30) Priority: 21.06.2019 EP 19181752
(43) Date of publication of application: 27.04.2022
(73) Proprietor: Unilever Global IP Limited, Wirral, Merseyside CH62 4ZD (GB); Unilever IP Holdings B.V., 6708 WH Wageningen (NL)
(72) Inventor: MOGHADAM, Arash, Mohajer, Wirral Merseyside CH63 3JW (GB); MUSCAT, Joseph, Wirral Merseyside CH63 3JW (GB); RILEY, Robert, George, Wirral Merseyside CH63 3JW (GB); STARCK, Pierre, Wirral Merseyside CH63 3JW (GB)
(74) Representative: Chisem, Janet
(86) International application number: PCT/EP2020/066619
(87) International publication number: WO 2020/254320

(56) References cited:
- WO-A1-2017/071915
- WO-A1-2018/007332
- WO-A2-2012/110387
- US-A1- 2011 002 868
- US-A1- 2012 076 747

## Description

### Field of the Invention

The present invention relates to shampoo compositions for use on hair.

### Background and Prior Art

US 2012/276210 relates to shampoo compositions containing polyacrylate microcapsules, wherein the polyacrylate microcapsules have increased deposition onto hair. A shampoo composition is disclosed comprising:
(a) from about 0.001% to about 10% of an anionic charged polyacrylate microcapsule;
(b) from about 0.01% to about 2% of a cationic deposition polymer; and
(c) from about 2% to about 25% of a detersive surfactant; and
(d) a carrier.

WO 2007/065537 addresses a problem associated with the use of cationic deposition polymers in that it is difficult to obtain a good balance of conditioning benefits at different stages of the shampooing process, and discloses an aqueous shampoo composition comprising:
(i) one or more anionic cleansing surfactants;
(ii) discrete, dispersed droplets of a water-insoluble conditioning agent with a mean droplet diameter (D3,2) of 4 micrometres or less;
(iii) one or more cationic polymers (A) selected from cationically modified acrylamide polymers having a cationic charge density at pH7 of less than 1.0 meq per gram, cationically modified celluloses and mixtures thereof, and
(iv) one or more cationic polymers (B) selected from cationically modified acrylamide polymers having a cationic charge density at pH7 of greater than 1.0 meq per gram, cationically modified polygalactomannans, and mixtures thereof, wherein the composition comprises a cationic polymer other than a cationically modified acrylamide polymer.

WO 2013/122861 discloses a conditioning composition additive for providing immediate and prolonged benefit to a keratin surface comprising:
a) a hydrophobically modified poly(acrylamido-N-propyltrimethylammonium chloride) (polyAPTAC) and b) water;
wherein the hydrophobically modified polyAPTAC is present in an amount of from 0.1 wt % to 20 wt % of the total weight of the conditioning composition additive and has a cationic charge density in the range of about 1 to 8 meq/g.

US20110002868 discloses a cationic polyelectrolyte formulation comprising a cationic synthetic water soluble polyelectrolyte, a surfactant and a solvent for use in personal care and household applications.

US2012/0076747 discloses a surfactant-based cleansing composition comprising, a surfactant, cationic water-soluble polyelectrolytes and the use of the composition in personal care and household care cleansing compositions for treating keratinous substrates, textile substance and hard-surface substrates.

WO2012110387 discloses cleansing compositions comprising two different cationic polymers, including a guar derivative, and a polysiloxane.

WO2018/007332 discloses personal cleansing composition comprising:_(i) an aqueous continuous phase including cleansing surfactant; (ii) one or more oily liquid conditioning agents for skin and/or hair wherein the agent is solubilized in wormlike micelles in the aqueous continuous phase via the incorporation of at least one inorganic electrolyte and at least one linker molecule; (iii) one or more cationic deposition polymers which are selected from cationic polygalactomannans having a mean charge density at pH7 from 0.2 to 2 meq per gram; and (iv) a hair substantive cationic conditioning polymer which is a homopolymer of (3-acrylamidopropyl) trimethyl ammonium chloride.

US20120076747 discloses personal cleansing compositions comprising anionic cleansing surfactants, an emulsified silicone, microcapsules containing a benefit agent, and a combination of cationic polymers composition a cationic polygalactomannan and an acrylaminopropyltrimonium chloride/acrylamide copolymer.

Many cleansing and conditioning products for use on hair contain silicones. It is desirable to deposit silicone onto hair in order to confer conditioning and sensory benefits. A typical hair wash process involves first washing hair with a shampoo and rinsing, followed by applying a conditioner product and rinsing.

Silicone can be deposited onto hair from a shampoo.

However, we have found that this silicone is largely deterged when the hair is subsequently washed with a conditioner as part of a typical washing process. A consequence of this is that it is necessary to include silicone in the conditioner in order to provide conditioning benefits that are apparent when the hair has dried.

We have now found that a shampoo comprising a conditioning polymer which is an APTAC polymer, preferably selected from a homopolymer of (3-acrylamidopropyl) trimethyl ammonium chloride and a (3-acrylamidopropyl) trimethyl ammonium chloride/acrylamide copolymer can enhance the adhesion of the silicone delivered from shampoo and help retain it on hair during and after washing with a conditioner.

When the hair is washed with a shampoo containing a conditioning polymer which is an APTAC polymer, preferably selected from a homopolymer of (3-acrylamidopropyl) trimethyl ammonium chloride and a (3-acrylamidopropyl) trimethyl ammonium chloride/acrylamide copolymer and then a silicone free conditioner, it has a significantly higher disposition of silicone compared to hair that is washed with a 1% silicone containing shampoo and then a silicone free conditioner.

In dry friction data, the hair that is washed with a shampoo containing a conditioning polymer which is an APTAC polymer, preferably selected from a homopolymer of (3-acrylamidopropyl) trimethyl ammonium chloride and a (3-acrylamidopropyl) trimethyl ammonium chloride/acrylamide copolymer and then a silicone free conditioner has a significantly lower dry friction compared to the hair that is washed with a 1% silicone containing shampoo and then a silicone free conditioner.

Use of such conditioning polymers in a shampoo composition having a combination of anionic and amphoteric surfactants at enriched amphoteric ratios, reduced surfactant concentrations and specific average SLES ethoxylation levels, gives excellent cleaning, deposition of benefit agents and desirable rheological and foaming characteristics, whilst maintaining mildness to skin and hair lipids and leaving hair feeling smooth and soft.

### Summary of the Invention

In a first aspect the present invention provides an aqueous shampoo for hair comprising:-
a. a pre-formed emulsified silicone;
b. from 0.05 to 2 wt % of a cationic deposition polymer, which is a cationic polygalactomannan having a mean charge density at pH7 of from 0.2 to 2 meq per gram;
c. from 0.05 to 5 wt % of a hair substantive cationic conditioning polymer which is an APTAC polymer having a molecular weight of less than 1 million Daltons and a charge density at pH 7 of from 4 to 6, selected from a homopolymer of (3-acrylamidopropyl) trimethyl ammonium chloride and a (3-acrylamidopropyl) trimethyl ammonium chloride/acrylamide copolymer;
d. from 3 to less than 12 wt %, preferably 5 to less than 10 wt %, based on total weight of the composition, of a cleansing surfactant, which has an average degree of ethoxylation of Eₙ, where n is a number that represents the average degree of ethoxylation and ranges from 0 to 3;
e. a co-surfactantwhich is a betaine surfactant selected from an amido betaine amphoteric surfactant of general formula (II): 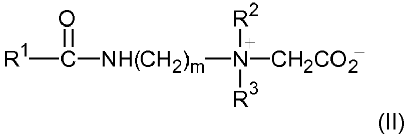 where m is 2 or 3; R¹C(O) is selected from linear or branched, saturated or unsaturated acyl groups having from 8 to 22 carbon atoms and mixtures thereof; and R² and R³ are each independently selected from alkyl, hydroxyalkyl or carboxyalkyl groups having from 1 to 6 carbon atoms and mixtures thereof;
   and an alkyl betaine of general formula (III): 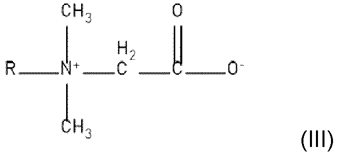 wherein R is a coco chain,
   and mixtures thereof;; and
f. a suspending agent
in which the weight ratio of (d) to (e) ranges from 1:1 to 4.5:1 and the pH of the composition is from 3 to 6.5.

In a second aspect, the present invention provides a method of treating hair comprising the steps of applying to hair the composition of the first aspect and performing a first rinse with water.

Silicone is deposited onto the hair from the composition of the invention, during the method of the invention.

Preferably, the method comprises a subsequent steps of applying a conditioner composition and then performing a second rinse with water. Following these subsequent steps, the silicone that is deposited on the hair from the composition of the invention remains on the hair.

In a third aspect, the present invention provides a use of a hair substantive cationic conditioning polymer which is an APTAC polymer having a molecular weight of less than 1 million Daltons, and a charge density at pH 7 of from 4 to 6, preferably selected from a homopolymer of (3-acrylamidopropyl) trimethyl ammonium chloride and a (3-acrylamidopropyl) trimethyl ammonium chloride/acrylamide copolymer in shampoo to retain silicone on the hair.

Some or all of the silicone is retained on the hair following rinse with water, and/or following treatment with a hair conditioner and a second rinse. By "retained" on the hair is meant that the silicone remains on the hair.

### Detailed Description and Preferred Embodiments

### The Emulsified Silicone

The composition of the invention comprises a pre-formed emulsified silicone. Mixtures of emulsified silicones can be used.

Suitable silicones include polydiorganosiloxanes, in particular polydimethylsiloxanes which have the CTFA designation dimethicone. Also suitable for use compositions of the invention are polydimethyl siloxanes having hydroxyl end groups, which have the CTFA designation dimethiconol. Also suitable for use in compositions of the invention are silicone gums having a slight degree of cross-linking, as are described for example in WO 96/31188.

The viscosity of the emulsified silicone itself (not the emulsion or the final hair composition) is typically at least 10,000 cst at 25 °C the viscosity of the silicone itself is preferably at least 60,000 cst, most preferably at least 500,000 cst, ideally at least 1,000,000 cst. Preferably the viscosity does not exceed 10⁹ cst for ease of formulation.

Emulsified silicones for use in the compositions of the invention will typically have a D90 silicone droplet size in the composition of less than 30, preferably less than 20, more preferably less than 10 micron, ideally from 0.01 to 1 micron. Silicone emulsions having an average silicone droplet size (D50) of 0.15 micron are generally termed microemulsions.

Silicone particle size may be measured by means of a laser light scattering technique, for example using a 2600D Particle Sizer from Malvern Instruments.

Examples of suitable pre-formed emulsions include Xiameter MEM 1785 and microemulsion DC2-1865 available from Dow Corning. These are emulsions /microemulsions of dimethiconol. Cross-linked silicone gums are also available in a pre-emulsified form, which is advantageous for ease of formulation.

A further preferred class of emulsified silicones for inclusion in compositions of the invention are amino functional silicones. By "amino functional silicone" is meant a silicone containing at least one primary, secondary or tertiary amine group, or a quaternary ammonium group. Examples of suitable amino functional silicones include: polysiloxanes having the CTFA designation "amodimethicone".

Specific examples of amino functional silicones suitable for use in the invention are the aminosilicone oils DC2-8220, DC2-8166 and DC2-8566 (all ex Dow Corning).

Suitable quaternary silicone polymers are described in EP-A-0 530 974. A preferred quaternary silicone polymer is K3474, ex Goldschmidt.

Also suitable are emulsions of amino functional silicone oils with non ionic and/or cationic surfactant.

Pre-formed emulsions of amino functional silicone are also available from suppliers of silicone oils such as Dow Corning and General Electric. Specific examples include DC939 Cationic Emulsion and the non-ionic emulsions DC2-7224, DC2-8467, DC2-8177 and DC2-8154 (all ex Dow Corning).

Preferably, the silicone is selected from the group consisting of dimethicone, dimethiconol, amodimethicone and mixtures thereof. Also preferred are blends of amino-functionalised silicones with dimethicones.

The total amount of silicone, at 100 % activity, is preferably from 0.01 wt% to 10 %wt of the total composition more preferably from 0.1 wt% to 5 wt%, most preferably 0.5 wt% to 3 wt% is a suitable level,

### The deposition polymer

The composition of the invention includes a cationic deposition polymer which is selected from cationic polygalactomannans having a mean charge density at pH7 from 0.2 to 2 meq per gram. Such polymers may serve to enhance the delivery of conditioning agents from the composition to the skin and/or hair surface during consumer use, thereby improving the conditioning benefits obtained. Mixtures of cationic deposition polymers may be employed.

The term "charge density" in the context of this invention refers to the ratio of the number of positive charges on a monomeric unit of which a polymer is comprised to the molecular weight of the monomeric unit. The charge density multiplied by the polymer molecular weight determines the number of positively charged sites on a given polymer chain.

The polygalactomannans are polysaccharides composed principally of galactose and mannose units and are usually found in the endosperm of leguminous seeds, such as guar, locust bean, honey locust, flame tree, and the like. Guar flour is composed mostly of a galactomannan which is essentially a straight chain mannan with single membered galactose branches. The mannose units are linked in a 1-4-β-glycosidic linkage and the galactose branching takes place by means of a 1-6 linkage on alternate mannose units. The ratio of galactose to mannose in the guar polymer is therefore one to two.

Suitable cationic polygalactomannans for use in the invention include polygalactomannans, such as guars, and polygalactomannan derivatives, such as hydroxyalkyl guars (for example hydroxyethyl guars or hydroxypropyl guars), that have been cationically modified by chemical reaction with one or more derivatizing agents.

Derivatizing agents typically contain a reactive functional group, such as an epoxy group, a halide group, an ester group, an anhydride group or an ethylenically unsaturated group, and at least one cationic group such as a cationic nitrogen group, more typically a quaternary ammonium group. The derivatization reaction typically introduces lateral cationic groups on the polygalactomannan backbone, generally linked via ether bonds in which the oxygen atom corresponds to hydroxyl groups on the polygalactomannan backbone which have reacted.

Preferred cationic polygalactomannans for use in the invention include guar hydroxypropyltrimethylammonium chlorides.

Guar hydroxypropyltrimethylammonium chlorides for use in the invention are generally comprised of a nonionic guar gum backbone that is functionalized with ether-linked 2-hydroxypropyltrimethylammonium chloride groups, and are typically prepared by the reaction of guar gum with N-(3-chloro-2-hydroxypropyl) trimethylammonium chloride.

Cationic polygalactomannans for use in the invention (preferably guar hydroxypropyltrimethylammonium chlorides) generally have an average molecular weight (weight average molecular mass (Mw) determined by size exclusion chromatography) in the range 500,000 to 3 million g/mol, more preferably 800,000 to 2.5 million g/mol.

Cationic polygalactomannans for use in the invention generally have a charge density ranging from 0.5 to 1.8 meq/g.

Preferably the cationic polygalactomannans are selected from guar hydroxypropyltrimethylammonium chlorides having a charge density ranging from 0.5 to 1.8 meq/g (and mixtures thereof).

The cationic charge density of the polymer is suitably determined via the Kjeldahl method as described in the US Pharmacopoeia under chemical tests for nitrogen determination.

Specific examples of preferred cationic polygalactomannans are guar hydroxypropyltrimonium chlorides having a cationic charge density from 0.5 to 1.1 meq/g.

Also suitable are mixtures of cationic polygalactomannans in which one has a cationic charge density from 0.5 to 1.1 meq/g, and one has a cationic charge density from 1.1 to 1.8 meq per gram.

Specific examples of preferred mixtures of cationic polygalactomannans are mixtures of guar hydroxypropyltrimonium chlorides in which one has a cationic charge density from 0.5 to 1.1 meq/g, and one has a cationic charge density from 1.1 to 1.8 meq per gram.

Cationic polygalactomannans for use in the invention are commercially available from Rhodia as JAGUAR ^{®} C13S, JAGUAR ^{®} C14 and JAGUAR ^{®} C17.

In the composition according to the invention the level of cationic polygalactomannans will range from 0.05 to 2%, preferably from 0.1 to 0.5, most preferably from 0.15 to 0.2% by weight based on the total weight of the composition.

In a preferred composition according to the invention the cationic polygalactomannans are selected from guar hydroxypropyltrimethylammonium chlorides having a charge density ranging from 0.5 to 1.8 meq/g (and mixtures thereof), at a level ranging from 0.15 to 0.2% by weight based on the total weight of the composition.

### The hair substantive cationic conditioning polymer

The composition of the invention includes a hair substantive cationic conditioning polymer which is an APTAC polymer having a molecular weight of less than 1 million Daltons, preferably selected from a homopolymer of (3-acrylamidopropyl) trimethyl ammonium chloride and a (3-acrylamidopropyl) trimethyl ammonium chloride/acrylamide copolymer.

WO2013/122861 describes the synthesis of (3-acrylamidopropyl) trimethyl ammonium chloride (APTAC) homopolymers of varying molecular weights, using a radical polymerisation reaction. According to the described method, APTAC monomer is polymerised in an aqueous medium by a discontinuous adiabatic process using an azo or persulfate radical initiator. The APTAC homopolymers so obtained have molecular weights ranging from about 100,000 g/mol to about 1,000,000 g/mol. The molecular weight can be determined by using standard analytical measurements, such as size exclusion chromatography (SEC).

A polymer suitable for use in the invention is commercially available from Ashland, Inc. as N-DurHance^{™} A-1000 Conditioning Polymer (supplied as a 20% a.i. aqueous solution of the polymer). A suitable copolymer of (3-acrylamidopropyl) trimethyl ammonium chloride/acrylamide copolymer is available from Ashland as N-DurHance AA2000.

The APTAC polymer for use in the invention, has a charge density at pH 7 of from 4 to 6.

The APTAC polymer for use in the invention has a molecular weight of less than 1 million Daltons, more preferably 100,000 to 950,000 Daltons, most preferably from 200,000 to 900,000 Daltons.

In a preferred embodiment the APTAC polymer has a charge density at pH 7 of from 4 to 6 and a molecular weight of 100,000 to 950,000 Daltons.

Suitable methods of measuring charge density and molecular weight are as given above.

In the composition according to the invention the level of polymer (*per* se as active ingredient) ranges from 0.05 to 5 %, more preferably from 0.1 to 2%, most preferably from 0.15 to 1 % (by weight based on the total weight of the composition).

### The cleansing surfactant

The composition of the invention comprises from 3 to less than 12 wt %, preferably 5 to less than 10 wt %, based on total weight of the composition, of a cleansing surfactant, which has an average degree of ethoxylation of Eₙ, where n is a number that represents the average degree of ethoxylation and ranges from 0 to 3.

The cleansing surfactant may suitably be selected from one or more anionic surfactants.

Typical anionic surfactants for use as cleansing surfactants in the invention include those surface active agents which contain an organic hydrophobic group with from 8 to 14 carbon atoms, preferably from 10 to 14 carbon atoms in their molecular structure; and at least one water-solubilising group which is preferably selected from sulphate, sulphonate, sarcosinate and isethionate.

Specific examples of such anionic surfactants include ammonium lauryl sulphate, ammonium laureth sulphate, trimethylamine lauryl sulphate, trimethylamine laureth sulphate, triethanolamine lauryl sulphate, trimethylethanolamine laureth sulphate, monoethanolamine lauryl sulphate, monoethanolamine laureth sulphate, diethanolamine lauryl sulphate, diethanolamine laureth sulphate, lauric monoglyceride sodium sulphate, sodium lauryl sulphate, sodium laureth sulphate, potassium lauryl sulphate, potassium laureth sulphate, sodium lauryl sarcosinate, sodium lauroyl sarcosinate, lauryl sarcosine, ammonium cocoyl sulphate, ammonium lauroyl sulphate, sodium cocoyl sulphate, sodium lauryl sulphate, potassium cocoyl sulphate, potassium lauryl sulphate, monoethanolamine cocoyl sulphate, monoethanolamine lauryl sulphate, sodium tridecyl benzene sulphonate, sodium dodecyl benzene sulphonate, sodium cocoyl isethionate and mixtures thereof.

Mixtures of any of the above described materials may also be used.

Preferably the cleansing surfactant is (i) one or more alkyl ether sulfate anionic surfactants of general formula (I)

R-O-(CH₂CH₂-O)ₙ-SO₃⁻M⁺ (I)

in which R is selected from linear or branched alkyl groups having from 10 to 14 carbon atoms and mixtures thereof; n is a number that represents the average degree of ethoxylation and ranges from 0 to 3, preferably 1 to 3, most preferably 1.5 to 2.5; and M is a solubilizing cation.

Preferably R in general formula (I) is a C₁₀ or C₁₂ linear alkyl group.

Preferably M in general formula (I) is selected from alkali metal cations (such as sodium or potassium), ammonium cations and substituted ammonium cations (such as alkylammonium, alkanolammonium or glucammonium).

Commercially produced alkyl ether sulfate anionic surfactants of general formula (I) may be made by sulfating fatty alcohol ethoxylates formed by reaction of ethylene oxide with fatty alcohol of formula R-OH (where R is as defined above). The reaction of the fatty alcohol with ethylene oxide typically yields mixtures of homologues which are alcohol polyethylene glycol ethers. Unreacted fatty alcohol may also be present in the mixture.

The distribution curve of the homologue mixture normally shows a maximum in the range from n-3 to n+3, where n denotes the average degree of ethoxylation in general formula (I). The value of n in general formula may be an integer or fraction, and may governed by factors such as the starting molar ratio of ethylene oxide to fatty alcohol in the reaction mixture, and the temperature, time and catalytic conditions under which the reaction takes place. Average n ranges from 0 to 3, preferably from 1 to 3, most preferably from 1.5 to 2.5. Blends of materials having different ethoxylation levels can be used to achieve an average degree of ethoxylation within the range.

Particularly preferred is SLES with an average of 2EO (i.e. sodium lauryl ether sulfate in which the average degree of ethoxylation n is 2.0). A suitable example of such a material is TEXAPON^{®} N 70 (ex BASF). A further example is sodium pareth ether sulphate, preferably with an average of 2EO.

All amounts referred to herein are based on 100 % activity unless otherwise stated.

All amounts referred to herein are based on 100 % activity (or "active") unless otherwise stated. By 100 % activity (or "active") is meant that the material is not diluted and is at 100 % v/v or wt/wt. Many materials used in personal care formulations are commercially available at different active concentrations, for example at 70 % active or 60 % active. For example, 100 ml of 70 % active surfactant provides the same amount of active material as 70 ml of 100 % active surfactant. Therefore, in order to provide for variations in activities of materials, all amounts are based on 100 % active materials.

### The co- surfactant

The composition of the invention comprises a co-surfactant.

Preferably the co-surfactant is selected from an amphoteric surfactant and a zwitterionic surfactant, most preferably an amphoteric surfactant.

Preferably, the co-surfactant is a betaine surfactant selected from an amido betaine amphoteric surfactant of general formula (II): 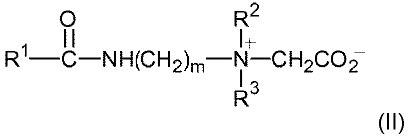
where m is 2 or 3; R¹C(O) is selected from linear or branched, saturated or unsaturated acyl groups having from 8 to 22 carbon atoms and mixtures thereof; and R² and R³ are each independently selected from alkyl, hydroxyalkyl or carboxyalkyl groups having from 1 to 6 carbon atoms and mixtures thereof;
an alkyl betaine of general formula (III): 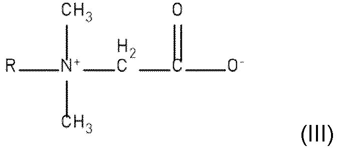 wherein R is a cocoyl group,
and mixtures thereof.

Preferably, R¹C(O) in general formula (II) is selected from linear acyl groups having from C₈ to C₁₈ carbon atoms and 0, 1, 2 or 3 double bonds and mixtures thereof.

More preferably, R¹C(O) in general formula (II) is selected from lauroyl, myristoyl, palmitoyl, stearoyl, oleoyl and cocoyl groups and mixtures thereof. Most preferably R¹C(O) in general formula (II) is a cocoyl group.

Preferably R² and R³ in general formula (II) are both methyl.

Mixtures of any of the above described materials may also be used.

The amount of amido betaine amphoteric surfactants of general formula (II) and (III) preferably ranges from 1 to 3.5 wt %, more preferably from 1 to 3 wt %, most preferably from 1.5 to 2.5 wt % (based on the total weight of the composition).

In a preferred composition according to the invention the amido betaine amphoteric surfactant of general formula (II) is cocamidopropylbetaine, in an amount ranging from 1 to 3% (by weight based on the total weight of the composition).

R in general formula (III) is a cocoyl group. This is preferably a blend of carbon chains resulting in an average carbon chain length of 12.

The combined amount of (i) and (ii) ranges from 5 to 10 wt %, preferably from 5 to 9 wt % (based on the total weight of the composition).

Preferably the weight ratio of the alkyl ether sulfate anionic surfactant (i) to the amido betaine amphoteric surfactant (ii) ranges from 1:1 to 4:1 [4.5:1?], more preferably from 1.5:1 to 3.75:1 and most preferably 2:1 to 3.5:1.

An especially preferred composition according to the invention comprises (d) SLES 2EO in an amount ranging from 3 to less than 7 wt % (by weight based on the total weight of the composition and 100 % active material); and (e) cocamidopropylbetaine in an amount ranging from 1 to 3 wt % (by weight based on the total weight of the composition and 100 % active material).

### The Suspending agent

The composition of the invention includes one or more suspending agents. Suitable suspending agents are selected from polyacrylic acids, cross-linked polymers of acrylic acid, copolymers of acrylic acid with a hydrophobic monomer, copolymers of carboxylic acid-containing monomers and acrylic esters, cross-linked copolymers of acrylic acid and acrylate esters, heteropolysaccharide gums and long chain acyl derivatives. The long chain acyl derivative is desirably selected from ethylene glycol stearate, alkanolamides of fatty acids having from 16 to 22 carbon atoms and mixtures thereof. Ethylene glycol distearate and polyethylene glycol 3 distearate are preferred long chain acyl derivatives, since these impart pearlescence to the composition. Polyacrylic acid is available commercially as Carbopol 420, Carbopol 488 or Carbopol 493. Polymers of acrylic acid cross-linked with a polyfunctional agent may also be used; they are available commercially as Carbopol 910, Carbopol 934, Carbopol 941 and Carbopol 980. An example of a suitable copolymer of a carboxylic acid containing monomer and acrylic acid esters is Carbopol 1342. All Carbopol (trademark) materials are available from Goodrich.

Suitable cross-linked polymers of acrylic acid and acrylate esters are Pemulen TR1 or Pemulen TR2. A suitable heteropolysaccharide gum is xanthan gum, for example that available as Kelzan mu.

Mixtures of any of the above suspending agents may be used. Preferred is a mixture of cross-linked polymer of acrylic acid and long chain acyl derivative.

The suspending agent will generally be present in a shampoo composition for use in the invention at levels of from 0.1 to 10%, preferably from 0.15 to 6%, more preferably from 0.2 to 4% by total weight of suspending agent based on the total weight of the composition

The aqueous composition of the invention suitably comprises from about 50 to about 90%, preferably from about 55 to about 85%, more preferably from about 60 to about 85%, most preferably from about 65 to about 83% water (by weight based on the total weight of the composition).

A preferred component of the composition is an inorganic electrolyte. Suitable inorganic electrolytes for use in the invention include metal chlorides (such as sodium chloride, potassium chloride, calcium chloride, magnesium chloride, zinc chloride, ferric chloride and aluminium chloride) and metal sulphates (such as sodium sulphate and magnesium sulphate). The inorganic electrolyte is used to assist in the solubilisation of oily components and to provide viscosity to the composition.

Examples of preferred inorganic electrolytes for use in the invention include sodium chloride, potassium chloride, magnesium sulphate and mixtures thereof.

Mixtures of any of the above described materials may also be suitable.

When included, the level of inorganic electrolyte in compositions of the invention generally ranges from about 1 to about 25%, preferably from about 1.5 to about 20% (by total weight inorganic electrolyte based on the total weight of the composition).

The composition of the invention may suitably have a viscosity ranging from 3,000 to 10,000 mPa.s, preferably from 4,000 to 9,000 mPa.s when measured using a Brookfield V2 viscometer (spindle RTV5, 1 minute, 20rpm) at 30°C.

A composition according to the invention may contain further optional ingredients to enhance performance and/or consumer acceptability. Examples of such ingredients include fragrance, dyes and pigments and pH adjusting agents. Each of these ingredients will be present in an amount effective to accomplish its purpose. Generally, these optional ingredients are included individually at a level of up to 5% by weight based on the total weight of the composition.

The pH of the composition of the invention suitably ranges from 3.0 to 7.0, and preferably ranges from 3.0 to 6.5, more preferably from 4 to 5.1.

The composition of the invention is primarily intended for topical application to the hair and scalp.

Most preferably the composition of the invention is topically applied to the hair and then massaged into the hair and scalp. The composition is then rinsed off the hair and scalp with water prior to drying the hair.

The invention will be further illustrated by the following, non-limiting Examples.

### EXAMPLES

### Example 1: Compositions 1-2, in accordance with the invention and Comparative Compositions A-B

Hair cleansing shampoo formulations were prepared, having ingredients as shown in Table 1. Compositions 1-2 are in accordance with the invention; Compositions A-B are comparative examples. All weight percentages (wt%) quoted are by weight based on total weight unless otherwise stated.

**Table 1: Compositions (wt %) of Compositions 1-2, in accordance with the invention, Comparative Compositions A-B**

| **INGREDIENT** | **COMPO SITION A** | **COMPO SITION 1** | **COMPO SITION B** | **COMPO SITION 2** |
|---|---|---|---|---|
| | **wt%** | | | |
| Sodium laureth sulphate (1EO) | 6.8 | 6.8 | 6 | 6 |
| Cocamidopropyl betaine (CAPB) | 3.2 | 3.2 | 2 | 2 |
| Guar hydroxypropyl trimonium chloride | 0.2 | 0.2 | 0.2 | 0.2 |
| Dimethiconol* | 3 | 3 | 3 | 3 |
| Carbomer | 0.4 | 0.4 | 0.4 | 0.4 |
| Cationic conditioning polymer ** | - | 1 | - | 1 |
| Sodium chloride | 0.2 | 0.2 | 0.2 | 0.2 |
| Water, and minors (perfume, preservatives, pH adjusters) | to 100% | to 100% | to 100% | to 100% |

| | | | | |
|---|---|---|---|---|
| * Emulsion of dimethiconol with anionic emulsifier, average particle size < 1 micron (ex Dow) ** N-DurHance^{™} A-1000 conditioning polymer (ex Ashland Inc.); 20 % active | | | | |

The shampoos Compositions 1-2 and Comparative Compositions A-B were prepared by the following method:
1. The cationic conditioning polymer was thoroughly dispersed in water.
2. The CAPB was then added.
3. The cleansing surfactant was added to the aqueous mixture and fully dispersed.
4. The suspending agent (carbomer) was added.
5. The guar polymer was dispersed in water and added to the mixture.
6. The silicone was then added with stirring and
7. The remaining minor ingredients were added.
8. Finally, the pH and viscosity of the shampoo were adjusted using pH adjuster (for example, citric acid) and sodium chloride respectively.

### Example 2: Treatment of hair with Compositions 1-2 and Comparative Compositions A-B

The hair used was dark brown European hair, in switches of 2.5 g weight and 6 inch length. This is referred to in these examples as Virgin hair.

Bleached hair was prepared as follows:
Hair was bleached once for 30 min with Platine Precision White Compact Lightening Powder (L'Oreal Professionnel Paris, Paris, France) mixed with 9% cream peroxide, 30 'vol' (Excel GS Ltd, UK) (60g of powder mixed with 120g cream peroxide). Hair was then rinsed with water for 2 minutes.

The formulations described in Table 1 were used to treat the hair during a typical washing protocol. The treated hair was then assessed for the level of silicone deposited onto the surface as well as the level of friction when the hair was dry.

Hair was treated with shampoo Compositions 1-2 and Comparative Compositions A-B using the following method:-
The hair fibres were held under running water for 30 seconds, shampoo applied at a dose of 0.1 ml of shampoo per 1g of hair and rubbed into the hair for 30 seconds. Excess lather was removed by holding under running water for 30 seconds and the shampoo stage repeated. The hair was rinsed under running water for 1 minute.

Hair was then treated with a silicone free conditioner where 0.2 ml conditioner per gram hair was applied and rubbed in for 1 min, then rinsed for 1 min under running water.

### Example 3: Silicone Deposition onto hair treated with Compositions 1-2 and Comparative Compositions A-B

Treated hair switches were rinsed and dried before the level of silicone deposited on the hair surface was quantified using x-ray fluorescence (XRF).

Five replicas were produced for each test formulation. The average amount of silicone deposited onto hair is shown in Table 2.

**Table 2: Deposition on silicone (ppm) onto bleached hair treated with Compositions 1-2, in accordance with the invention, Comparative Compositions A-B**

| **Composition** | **Hair type** | **Silicone Deposition (ppm)** | **s.d.** |
|---|---|---|---|
| Composition A | bleached | 406 | 124.85 |
| Composition 1 | bleached | 888 | 97.24 |
| Composition B | bleached | 978 | 131.94 |
| Composition 2 | bleached | 1469 | 126.35 |

It will be seen that the level of silicone deposited onto hair is dramatically higher for Compositions 1 and 2, in accordance with the invention, than for the comparative Compositions A and B, which did not comprise the polymer.

### Example 4: Friction of hair treated with Compositions 1-2 and Comparative Compositions A-B

In a further series of tests, formulations described in Table 1 were assessed for the level of friction following a typical hair washing protocol on switches of virgin or once-bleached dark brown European (DBE) hair. Five replicas were produced for each test formulation. The average of measured friction is shown in Table 3.

Hair was first washed with shampoo and conditioner as in Example 2 above.

The friction of the dry hair was assessed using a Texture Analyser fitted with a 500 g weight on top of the probe.

**Table 3: Friction of bleached hair treated with Compositions 1-2, in accordance with the invention and Comparative Compositions A-B**

| **Formulation** | **Dry Friction (mm.g)** | **s.d.** |
|---|---|---|
| Composition A | 32217 | 4956.84 |
| Composition 1 | 23516 | 911.85 |
| Composition B | 25395 | 2868.56 |
| Composition 2 | 20883 | 769.75 |

It can be seen that hair treated with Compositions in accordance with the invention produces less friction than hair treated with Comparative Compositions A and B.

## Claims

1. An aqueous shampoo composition comprising:-
a. a pre-formed emulsified silicone;
b. from 0.05 to 2 wt % of a cationic deposition polymer, which is a cationic polygalactomannan having a mean charge density at pH7 of from 0.2 to 2 meq per gram;
c. from 0.05 to 5 wt % of a hair substantive cationic conditioning polymer which is an APTAC polymer having a molecular weight of less than 1 million Daltons, and a charge density at pH 7 of from 4 to 6, selected from a homopolymer of (3-acrylamidopropyl) trimethyl ammonium chloride and a (3-acrylamidopropyl) trimethyl ammonium chloride/acrylamide copolymer;
d. from 3 to less than 12 wt %, preferably 5 to less than 10 wt %, based on total weight of the composition, of a cleansing surfactant, which has an average degree of ethoxylation of Eₙ, where n is a number that represents the average degree of ethoxylation and ranges from 0 to 3;
e. a co-surfactant, which is a betaine surfactant selected from an amido betaine amphoteric surfactant of general formula (II): 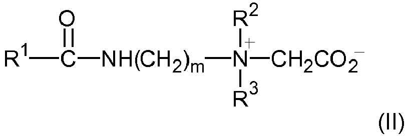 where m is 2 or 3; R¹C(O) is selected from linear or branched, saturated or unsaturated acyl groups having from 8 to 22 carbon atoms and mixtures thereof; and R² and R³ are each independently selected from alkyl, hydroxyalkyl or carboxyalkyl groups having from 1 to 6 carbon atoms and mixtures thereof;
and an alkyl betaine of general formula (III): 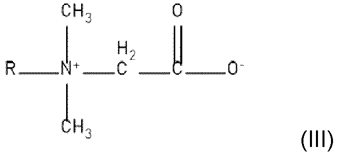
wherein R is a coco chain,
and mixtures thereof; and
f. a suspending agent.
in which the weight ratio of (d) to (e) ranges from 1:1 to 4.5:1 and the pH of the composition is from 3 to 6.5.

2. A composition as claimed in claim 1, wherein the amount of emulsified silicone is from 0.01 to 10 wt % based on the total weight of the composition and 100 % activity.

3. A composition as claimed in claim 1 or claim 2, wherein the the emulsified silicone is selected from the group consisting of dimethicone, dimethiconol, amodimethicone and mixtures thereof.

4. A composition as claimed in any preceding claim, wherein the cleansing surfactant is an alkyl ether sulfate anionic surfactant of general formula (I):
R-O-(CH₂CH₂-O)ₙ-SO₃⁻M⁺ (I)
in which R is selected from linear or branched alkyl groups having from 10 to 14 carbon atoms and mixtures thereof; n is a number that represents the average degree of ethoxylation and ranges from 0 to 3; and M is a solubilizing cation.

5. A composition as claimed in any preceding claim , wherein the amphoteric surfactants of general formula (II) and (III) are present in an amount of from 1 to 3.5 wt %, based on the total weight of the composition.

6. A composition according to any preceding claim, in which the cationic deposition polymer is a cationic polygalactomannan selected from guar hydroxypropyltrimethylammonium chlorides with a charge density ranging from 0.5 to 1.8 meq/g and mixtures thereof.

7. A composition according to claim 6, in which the level of the guar hydroxypropyltrimethylammonium chloride(s) ranges from 0.15 to 0.2% by weight based on the total weight of the composition.

8. A composition according to any preceding claim, in which the level of hair substantive cationic conditioning polymer, *per se* as active ingredient, ranges from 0.1 to 2%,by weight based on the total weight of the composition.

9. A composition as claimed in any preceding claim, wherein the suspending agent is selected from polyacrylic acids, cross-linked polymers of acrylic acid, copolymers of acrylic acid with a hydrophobic monomer, copolymers of carboxylic acid-containing monomers and acrylic esters, cross-linked copolymers of acrylic acid and acrylate esters, heteropolysaccharide gums and long chain acyl derivatives.

10. A composition as claimed in any preceding claim, wherein the APTAC polymer has a molecular weight of 100,000 to 950,000 Daltons.

11. A method of treating hair comprising the steps of applying to hair the composition as defined in any one of claims 1 to 10, and performing a first rinse with water.

12. A method as claimed in claim 11, which comprises the subsequent steps of applying a conditioner composition and performing a second rinse with water.

13. A use of a hair substantive cationic conditioning polymer which is an APTAC polymer having a molecular weight of less than 1 million Daltons, and a charge density at pH 7 of from 4 to 6, preferably selected from a homopolymer of 3-acrylamidopropyl trimethyl ammonium chloride and a 3-acrylamidopropyl trimethyl ammonium chloride/acrylamide copolymer in shampoo to retain silicone on the hair.

## Patentansprüche

1. Wässrige Shampoo-Zusammensetzung, umfassend:
a. ein vorgeformtes emulgiertes Silikon;
b. 0,05 bis 2 Gew.-% eines kationischen Ablagerungspolymers, das ein kationisches Polygalactomannan mit einer mittleren Ladungsdichte von 0,2 bis 2 meq/Gramm bei einem pH-Wert von 7 ist;
c. 0,05 bis 5 Gew.-% eines haarsubstantiven kationischen Konditionierungspolymers, das ein APTAC-Polymer mit einem Molekulargewicht von weniger als 1 Million Dalton und einer Ladungsdichte von 4 bis 6 bei einem pH-Wert von 7 ist, ausgewählt unter einem Homopolymer von (3-Acrylamidopropyl)-trimethylammoniumchlorid und einem (3-Acrylamidopropyl)-trimethylammoniumchlorid/Acrylamid-Copolymer;
d. 3 bis weniger als 12 Gew.-%, vorzugsweise 5 bis weniger als 10 Gew.-%, bezogen auf das Gesamtgewicht der Zusammensetzung, eines reinigenden Tensids, das einen durchschnittlichen Ethoxylierungsgrad von Eₙ aufweist, wobei n eine Zahl ist, die den durchschnittlichen Ethoxylierungsgrad darstellt und in dem Bereich von 0 bis 3 liegt;
e. ein Co-Tensid, das ein Betain-Tensid ist, ausgewählt unter einem amphoteren Amidobetain-Tensid der allgemeinen Formel (II): 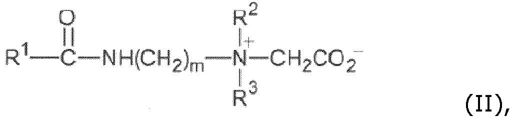 worin m 2 oder 3 ist;
R¹C(O) unter linearen oder verzweigten, gesättigten oder ungesättigten Acylgruppen mit 8 bis 22 Kohlenstoffatomen und Mischungen davon ausgewählt ist; und
R² und R³, jeweils unabhängig voneinander, unter Alkyl-, Hydroxyalkyl- oder Carboxylalkylgruppen mit 1 bis 6 Kohlenstoffatomen und Mischungen davon ausgewählt sind;
und einem Alkylbetain der allgemeinen Formel (III) 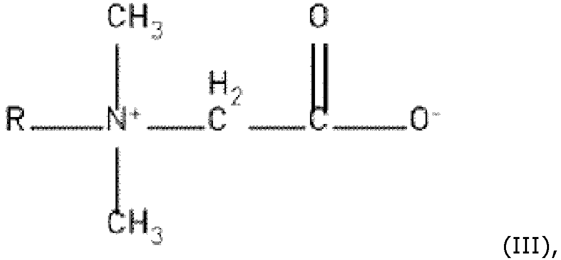
worin R eine Coco-Kette ist, und
Mischungen davon; und
f. ein Suspensionsmittel;
in der das Gewichtsverhältnis von (d) zu (e) in dem Bereich von 1:1 bis 4,5:1 und der pH-Wert der Zusammensetzung zwischen 3 und 6,5 liegt.

2. Zusammensetzung nach Anspruch 1, wobei die Menge an emulgiertem Silikon 0,01 bis 10 Gew.-%, bezogen auf das Gesamtgewicht der Zusammensetzung und 100% Aktivität, beträgt.

3. Zusammensetzung nach Anspruch 1 oder Anspruch 2, wobei das emulgierte Silikon aus der Gruppe, die aus Dimethicon, Dimethiconol, Amodimethicon und Mischungen davon besteht, ausgewählt ist.

4. Zusammensetzung nach einem vorhergehenden Anspruch, wobei das reinigende Tensid ein anionisches Alkylethersulfat-Tensid der allgemeinen Formel (I)
R-O-(CH₂CH₂-O)ₙ-SO₃⁻M⁺ (I)
ist,
worin R unter linearen oder verzweigten Alkylgruppen mit 10 bis 14 Kohlenstoffatomen und Mischungen davon ausgewählt ist,
n eine Zahl ist, die den durchschnittlichen Ethoxylierungsgrad darstellt und in dem Bereich von 0 bis 3 liegt; und
M ein solubilisierendes Kation ist.

5. Zusammensetzung nach einem vorhergehenden Anspruch, wobei die amphoteren Tenside der allgemeinen Formeln (II) und (III) in einer Menge von 1 bis 3,5 Gew.-%, bezogen auf das Gesamtgewicht der Zusammensetzung, vorliegen.

6. Zusammensetzung nach einem vorhergehenden Anspruch, bei der das kationische Ablagerungspolymer ein kationisches Polygalactomannan ist, das aus Guarhydroxypropyltrimethylammoniumchloriden mit einer Ladungsdichte in dem Bereich von 0,5 bis 1,8 meq/g und Mischungen davon ausgewählt ist.

7. Zusammensetzung nach Anspruch 6, bei der der Gehalt an Guarhydroxypropyltrimethylammoniumchlorid(en) in dem Bereich von 0,15 bis 0,2 Gew.-%, bezogen auf das Gesamtgewicht der Zusammensetzung, liegt.

8. Zusammensetzung nach einem der vorhergehenden Ansprüche, bei der der Gehalt an haarsubstantivem kationischem Konditionierungspolymer, per se als aktiver Bestandteil, in dem Bereich von 0,1 bis 2 Gew.-%, bezogen auf das Gesamtgewicht der Zusammensetzung, liegt.

9. Zusammensetzung nach einem vorhergehenden Anspruch, wobei das Suspensionsmittel unter Polyacrylsäuren, vernetzten Polymeren von Acrylsäure, Copolymeren von Acrylsäure mit einem hydrophoben Monomer, Copolymere von carbonsäurehaltigen Monomeren und Acrylestern, vernetzten Copolymeren von Acrylsäure und Acrylatestern, Heteropolysaccharidgummis und langkettigen Acylderivaten ausgewählt ist.

10. Zusammensetzung nach einem vorhergehenden Anspruch, wobei das APTAC-Polymer ein Molekulargewicht von 100.000 bis 950.000 Dalton aufweist.

11. Verfahren zur Behandlung von Haar, umfassend die Schritte des Auftragens der in irgendeinem der Ansprüche 1 bis 10 definierten Zusammensetzung auf das Haar und des Durchführens einer ersten Spülung mit Wasser.

12. Verfahren nach Anspruch 11, das die nachfolgenden Schritte des Auftragens einer Konditionierungszusammensetzung und des Durchführens einer zweiten Spülung mit Wasser umfasst.

13. Verwendung eines haarsubstantiven kationischen Konditionierungspolymers, das ein APTAC-Polymer mit einem Molekulargewicht von weniger als 1 Million Dalton und einer Ladungsdichte von 4 bis 6 bei einem pH-Wert von 7 ist, vorzugsweise ausgewählt unter einem Homopolymer von 3-Acrylamidopropyltrimethylammoniumchlorid und einem 3-Acrylamidopropyltrimethylammoniumchlorid/Acrylamid-Copolymer, in einem Shampoo, um Silikon auf dem Haar zu behalten.

## Revendications

1. Composition aqueuse de shampooing comprenant :
a. une silicone émulsionnée préformée ;
b. de 0,05 à 2 % en poids d'un polymère de dépôt cationique, qui est une polygalactomannane cationique ayant une densité de charge moyenne à pH 7 de 0,2 à 2 méq par gramme ;
c. de 0,05 à 5 % en poids d'un polymère conditionneur cationique substantif capillaire qui est un polymère APTAC ayant un poids moléculaire inférieur à 1 million de Daltons, et une densité de charge à pH 7 de 4 à 6, choisi parmi un homopolymère de chlorure de (3-acrylamidopropyl)triméthylammonium et un copolymère de chlorure de (3-acrylamidopropyl)triméthylammonium/acrylamide ;
d. de 3 à moins de 12 % en poids, de préférence de 5 à moins de 10 % en poids, par rapport au poids total de la composition, d'un tensioactif nettoyant qui a un degré moyen d'éthoxylation de Eₙ où n est un nombre qui représente le degré moyen d'éthoxylation et est situé dans la plage allant de 0 à 3 ;
e. un cotensioactif qui est un tensioactif bétaïne choisi parmi un tensioactif amphotère amidobétaïne de formule générale (II) : 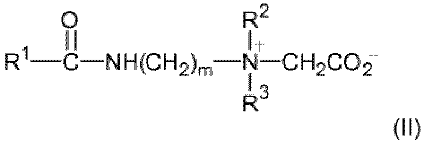
où m vaut 2 ou 3 ; R¹C(O) est choisi parmi les groupes acyle linéaires ou ramifiés, saturés ou insaturés, ayant de 8 à 22 atomes de carbone, et leurs mélanges ; et chacun de R² et R³ est indépendamment choisi parmi les groupes alkyle, hydroxyalkyle et carboxyalkyle ayant de 1 à 6 atomes de carbone, et leurs mélanges ;
et une alkylbétaïne de formule générale (III) : 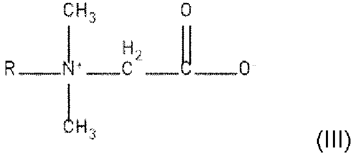
dans laquelle R est une chaîne coprah-yle,
et leurs mélanges ; et
f. un agent de mise en suspension,
dans laquelle le rapport en poids de (d) à (e) est situé dans la plage allant de 1/1 à 4,5/1 et le pH de la composition est de 3 à 6,5.

2. Composition selon la revendication 1, dans laquelle la quantité de silicone émulsionnée est de 0,01 à 10 % en poids par rapport au poids total de la composition et pour une activité de 100 %.

3. Composition selon la revendication 1 ou la revendication 2, dans laquelle la silicone émulsionnée est choisie dans le groupe constitué par la diméthicone, le diméthiconol, l'amodiméthicone et leurs mélanges.

4. Composition selon l'une quelconque des revendications précédentes, dans laquelle le tensioactif nettoyant est un tensioactif anionique alkyléthersulfate de formule générale (I) :
R-O-(CH₂CH₂-O)ₙ-SO₃⁻M⁺ (I)
dans laquelle R est choisi parmi les groupes alkyle linéaires et ramifiés ayant de 10 à 14 atomes de carbone et leurs mélanges ; n est un nombre qui représente le degré moyen d'éthoxylation et est situé dans la plage allant de 0 à 3 ; et M est un cation solubilisant.

5. Composition selon l'une quelconque des revendications précédentes, dans laquelle les tensioactifs amphotères de formules générales (II) et (III) sont présents en une quantité de 1 à 3,5 % en poids par rapport au poids total de la composition.

6. Composition selon l'une quelconque des revendications précédentes, dans laquelle le polymère de dépôt cationique est une polygalactomannane cationique choisie parmi les chlorures d'hydroxypropyltriméthylammonium-guar ayant une densité de charge située dans la plage allant de 0,5 à 1,8 méq/g et leurs mélanges.

7. Composition selon la revendication 6, dans laquelle le taux du ou des chlorures d'hydroxypropyltriméthylammonium-guar est situé dans la plage allant de 0,15 à 0,2 % en poids par rapport au poids total de la composition.

8. Composition selon l'une quelconque des revendications précédentes, dans laquelle le taux de polymère conditionneur cationique substantif capillaire, en tant qu'ingrédient actif *per se,* est situé dans la plage allant de 0,1 à 2 % en poids par rapport au poids total de la composition.

9. Composition selon l'une quelconque des revendications précédentes, dans laquelle l'agent de mise en suspension est choisi parmi les poly(acides acryliques), les polymères réticulés d'acide acrylique, les copolymères d'acide acrylique avec un monomère hydrophobe, les copolymères de monomères contenant de l'acide carboxylique et d'esters acrylates, les copolymères réticulés d'acide acrylique et d'esters acrylate, les gommes d'hétéropolysaccharides et les dérivés acyle à longue chaîne.

10. Composition selon l'une quelconque des revendications précédentes, dans laquelle le polymère APTAC a un poids moléculaire de 100 000 à 950 000 Daltons.

11. Méthode de traitement capillaire, comprenant les étapes d'application aux cheveux de la composition telle que définie dans l'une quelconque des revendications 1 à 10, et de réalisation d'un premier rinçage à l'eau.

12. Méthode selon la revendication 11, qui comprend les étapes subséquentes d'application d'une composition de conditionneur et de réalisation d'un deuxième rinçage à l'eau.

13. Utilisation d'un polymère conditionneur cationique substantif capillaire qui est un polymère APTAC ayant un poids moléculaire inférieur à 1 million de Daltons, et une densité de charge à pH 7 de 4 à 6, de préférence choisi parmi un homopolymère de chlorure de (3-acrylamidopropyl)triméthyl-ammonium et un copolymère de chlorure de (3-acrylamidopropyl)triméthylammonium/acrylamide, dans un shampooing pour retenir la silicone sur les cheveux.
